# EUROPÄISCHE PATENTANMELDUNG

(11) **EP 4 380 318 A1**
(43) Veröffentlichungstag der Anmeldung: **05.06.2024**
(21) Anmeldenummer: 22210434.1
(22) Anmeldetag: 30.11.2022
(51) Int. Cl.: H05H 1/24, A61L 9/22, B60H 3/00

(54) **PLASMA-FILTEREINRICHTUNG, ELEKTRODENEINRICHTUNG UND VERFAHREN ZUM BETREIBEN EINER PLASMA-FILTEREINRICHTUNG**

(71) Anmelder: Siemens Healthineers AG, 91301 Forchheim (DE)
(72) Erfinder: Feher, Lambert, 76297 Stutensee (DE); Höcht, Philipp, 91207 Lauf (DE); Meise, Florian, 91353 Hausen (DE); Model, Volker, 90766 Fürth (DE); Seifert, Martin, 95447 Bayreuth (DE)
(74) Vertreter: Siemens Healthineers Patent Attorneys

(57) **Zusammenfassung**

Die Erfindung betrifft eine Plasma-Filtereinrichtung (1), aufweisend zumindest eine Elektrodeneinrichtung (2). Es ist vorgesehen, dass die Elektrodeneinrichtung (2) eine erste plan ausgebildete Kompositelektrode (3) und eine zweite plan ausgebildete Kompositelektrode (4) aufweist. Die Kompositelektroden (3, 4) der Elektrodeneinrichtung (2) sind coplanar zueinander in einer Hauptflächenebene (13) der Elektrodeneinrichtung (2) angeordnet und durch einen Entladungsspalt (9) räumlich voneinander getrennt. Jede der Kompositelektroden (3, 4) weist ein jeweiliges Elektrodenblech (5, 6) auf, das zumindest an einer Grenzfläche des jeweiligen Elektrodenblechs (5, 6) zu dem Entladungsspalt (9) eine jeweilige dielektrische Beschichtung (7, 8) aufweist. Die Plasma-Filtereinrichtung (1) weist eine Spannungsquelle (22) auf, die dazu eingerichtet ist, eine Wechselspannung an der Elektrodeneinrichtung (2) bereitzustellen, wobei die Wechselspannung dazu parameterisiert ist, eine Bildung eines Plasmas (11) durch eine dielektrische Barriereentladung in dem Entladungsspalt (9) hervorzurufen.

## Beschreibung

*Unabhängig vom grammatikalischen Geschlecht eines bestimmten Begriffes sind Personen mit männlicher, weiblicher oder anderer Geschlechteridentität mit umfasst.*

Die vorliegende Erfindung betrifft eine Plasma-Filtereinrichtung, aufweisend zumindest eine Elektrodeneinrichtung, eine Elektrodeneinrichtung sowie ein Verfahren zum Betreiben einer Plasma-Filtereinrichtung.

Schon vor der Coronavirus-Pandemie war die Verhinderung der Übertragung von Krankheitserregern durch Aerosole eine Herausforderung für die Krankenhaushygiene. Bisher wurde die Reduktion von Keimen und Partikeln nur in speziellen Räumen wie Operationssaal, Labor, Isolationsbereich durchgeführt. Aufgrund der Pandemie gewinnt die Forderung nach einer pathogenfreien Lüftung jedoch in Kombination mit Lüftungs- und Klimaanlagen im Allgemeinen an Bedeutung. Aktuell existieren verschiedene marktverfügbare Filterlösungen auf Basis von Plasma, UVC-Strahlung und deren Kombinationen mit zusätzlichen Filtern wie zum Beispiel HEPA für die Aufbereitung von Raumluft in geschlossenen Umgebungen wie Gebäuden, Autos, Flugzeugen oder Zügen.

Aufgrund der sehr spezifischen Anforderung der verschiedenen Anwendungsmöglichkeiten haben marktverfügbare Lösungen Nachteile bezüglich der Strömungsmechanik des zu filternden Gases oder einer Baugröße der Filtervorrichtung. Zudem können bestehende Lösungen hohe Kosten verursachen, welche durch bereitzustellende Filtereinheiten und einem regelmäßigem Austausch und einer Entsorgung von zusätzlichen Filterelementen wie HEPA entstehen können.

Somit ist eine effektive sowie effiziente Einbringung in und Optimierung aktuell eingesetzter Filterlösungen nur bedingt oder unmöglich.

Bekannte Filtervorrichtungen nach dem Stand der Technik umfassen beispielsweise mechanische Luftfilter.

Aktuell ist die mechanische Filtration von Raumluft von großer Bedeutung, um die Raumluft sauber zu halten und zu sterilisieren, insbesondere in Krankenhäusern und anderen medizinischen Einrichtungen. Moderne Gebäudeluftreinigungssysteme verfügen über mehrstufige Filteranordnungen, die aus Vorfiltern für zum Beispiel Staub und hocheffizienten Filtern zum Beispiel für mikrobielle Kontamination bestehen. In OP-Anwendungen werden diese mit mechanischen Vorrichtungen kombiniert, um einen laminaren (turbulenzfreien) Luftstrom über dem Patienten zu erzeugen. In heutigen Operationssaalinstallationen (sog. Lüftungs- und Klimaanlagen) wird Frischluft zugeführt, die vor allem im Winter mit viel Energie auf die übliche Raumtemperatur (20-22 Grad) erwärmt werden muss.

Mit einem Abscheidegrad von mindestens 99,95% halten hocheffiziente HEPA-Filter Bakterien und Viren neben der Partikelkontamination effektiv zurück. SARS-CoV-2-Viren kommen jedoch auch in mesoskaligen Aerosolen vor, die diese Filter passieren können. Insbesondere diese feinen Aerosole haben eine lange Verweildauer in der Luft.

Für Gebäude wurde in der Pandemie im Jahr 2021 vorgeschlagen, die Belüftung mit HEPA-Filtern zu verbessern. Große und kostenintensive Anlagenerweiterungen sind die Folge. Die führt auch zu einer erheblichen Verschlechterung der Energiebilanz durch Kompensation von Druckverlusten sowie durch eine Erhöhung der Strömungsgeschwindigkeit in erhöhter Geräuschemission resultieren.

Für Flugzeuge werden zentrale Filteranlagen mit HEPA Einheiten verwendet, um die Luft zu reinigen. Die Größe, das Gewicht und der Energieverbrauch beeinflussen die Gesamtleistung eines Flugzeugs durch dieses Belüftungssystem. Automobil-/Bus-/Zugabteile bestehen nur aus zentralen Klimakammern mit einfacher Filtertechnik, die nicht geeignet ist, um eine Dekontamination der Luft zu ermöglichen.

Neben mechanischen Filtervorrichtungen werden auch Filtervorrichtungen angewandt, welche UVC- oder plasmabasierte Verfahren zur Dekontamination von Luft ermöglichen.

Gängige Ozonisatoren, Ionisatoren oder Plasmasysteme bestehen aus umfangreichen Wechselwirkungskammern. Um zum Beispiel Raumluft mit UV-Lampen zu dekontaminieren, finden tunnelartige Systeme mit langsamen bis mäßigen Luftströmungen Anwendung. Nur dadurch können hohe Dekontaminationsraten erzielt werden. Daher ist allen diesen Filtervorrichtungen gemeinsam, dass sie als zentrale Einheiten mit einem erweiterten würfelartigen oder räumlich länglichen Design verwendet werden.

Seit kurzem werden mobile Oberflächendekontaminationsgeräte oder stationäre UVC-Lampen zur Oberflächendekontamination eingesetzt. Ein Nachteil dieser marktverfügbaren Lösungen ist die Implementierung in zum Beispiel medizinische Bildgebungs-Geräte aufgrund der Anforderungen zum Beispiel in Bezug auf Bauraum, EMV oder der Strömungsverhältnisse.

Marktverfügbare Lösungen basieren auf elektromagnetischen Entladungen und einer vergleichsweise langsamen Desinfektionswirkung durch Radikale.

Weitergehende Lösungen kombinieren daher zusätzlich UVCbasierte Verfahren mit dem erzeugten Plasma. Bei UVCbasierten Verfahren ist die für eine effektive und effiziente Dekontamination der Luft erforderliche Dosisleistung stark von der Wellenlänge der verwendeten UVC-Quelle und der Verweilzeit der Raumluft in der entsprechenden Filtereinheit abhängig. Erfahrungen aus der Oberflächendekontamination durch UVC-Strahlung haben gezeigt, dass teilweise lange Bestrahlungszeiten in Abhängigkeit von der Entfernung und der Dosisleistung notwendig sind, um eine hohe Keimelimination zu gewährleisten.

In Bezug auf medizinische Geräte ist aus der CN203524686U eine CT-Maschine mit Selbstreinigungsfunktion bekannt. Hierbei ist eine Sterilisationseinheit auf Basis von UVC, Plasma oder Ozon für die CT-Maschine offenbart.

Es ist eine Aufgabe der Erfindung, eine Filtereinrichtung bereitzustellen, welche eine effizientere Dekontamination von Luft ermöglicht.

Diese Aufgabe wird gelöst durch den jeweiligen Gegenstand der unabhängigen Ansprüche. Vorteilhafte Weiterbildungen und bevorzugte Ausführungsformen sind Gegenstand der abhängigen Ansprüche.

Ein erster Aspekt Erfindung betrifft eine Plasma-Filtereinrichtung, die zumindest eine Elektrodeneinrichtung aufweist. Bei der Plasma-Filtereinrichtung handelt es sich um eine Filtereinrichtung zum Filtern eines Gases, beispielsweise zum Filtern von Luft, wobei zum Filtern des Gases ein Plasma erzeugt wird. Durch das erzeugte Plasma wird UV-Strahlung abgegeben, durch welche beispielsweise in dem Gas befindliche Aerosole oder Keime inaktiviert oder Partikel deaktiviert werden können.

Es ist vorgesehen, dass die Elektrodeneinrichtung eine erste plan ausgebildete Kompositelektrode und eine zweite plan ausgebildete Kompositelektrode aufweist. Die Kompositelektroden können beispielsweise einheitliche, symmetrische Elemente umfassen, die in antisymmetrischer elektromagnetischer Potentialkontur angeordnet sein können. Mit anderen Worten umfasst die Elektrodeneinrichtung die erste Kompositelektrode und die zweite Kompositelektrode. Die Kompositelektroden der Elektrodeneinrichtung können insbesondere als Flächenelemente eingerichtet sein. Es ist vorgesehen, dass die Kompositelektroden der Elektrodeneinrichtung coplanar zueinander in einer Hauptflächenebene der Elektrodeneinrichtung angeordnet und durch einen Entladungsspalt räumlich voneinander getrennt sind. Mit anderen Worten befinden sich die Kompositelektroden der Elektrodeneinrichtung in der Hauptflächenebene der Elektrodeneinrichtung. Zwischen den Kompositelektroden der Elektrodeneinrichtung befindet sich ein Entladungsspalt. Bei dem Entladungsspalt handelt es sich um einen durch die Kompositelektroden gebildeten Spalt der Elektrodeneinrichtung zum Durchleiten des zu filternden Gases. Es ist vorgesehen, dass jede der Kompositelektroden ein jeweiliges Elektrodenblech aufweist, das zumindest an einer Grenzfläche des jeweiligen Elektrodenblechs zu dem Entladungsspalt eine jeweilige dielektrische Beschichtung aufweist. Mit anderen Worten sind die Elektroden als Komposite bereitgestellt, welche das jeweilige Elektrodenblech und eine auf dem jeweiligen Elektrodenblech befindliche dielektrische Beschichtung umfassen. Die dielektrische Beschichtung ist zumindest an einer von der jeweiligen Kompositelektrode an dem Entladungsspalt angrenzende Grenzfläche auf dem Elektrodenblech aufgetragen.

Die Plasma-Filtereinrichtung weist eine Spannungsquelle auf, die dazu eingerichtet ist, eine Wechselspannung an der Elektrodeneinrichtung bereitzustellen wobei die Wechselspannung dazu parameterisiert ist, eine Bildung eines Plasmas durch eine dielektrische Barriereentladung in dem Entladungsspalt hervorzurufen. Mit anderen Worten ist es vorgesehen, dass die Plasma-Filtereinrichtung die Spannungsquelle aufweist. Die Spannungsquelle ist dazu vorgesehen, die Wechselspannung an der Elektrodeneinrichtung bereitzustellen, um die Bildung des Plasmas in dem Entladungsspalt hervorzurufen.

Die Plasma-Filtereinrichtung ist dazu eingerichtet, ein Gas entlang einer Hauptströmungsrichtung, welche parallel zu einer Normalen der Hauptflächenebene der Elektrodeneinrichtung ausgerichtet ist, durch den Entladungsspalt zu führen. Mit anderen Worten weist die Plasma-Filtereinrichtung strömungstechnische Führungselemente, beispielsweise Röhrenelemente auf, die dazu eingerichtet sind, eine Hauptströmungsrichtung des Gases der Art zu beeinflussen oder festzulegen, dass das Gas parallel zu der Normalen der Hauptflächenebene durch den Entladungsspalt geleitet wird.

Die Hauptströmungsrichtung verläuft dabei parallel zu der Normalen der Hauptflächenebene der Elektrodeneinrichtung. Mit anderen Worten verläuft die Hauptströmungsrichtung senkrecht zu der Elektrodeneinrichtung. Das Gas strömt somit senkrecht durch die Elektrodeneinrichtung durch den Entladungsspalt. Aufgrund des in dem Entladungsspalt durch die dielektrische Entladung erzeugten Plasmas, erfolgt eine Dekontamination des Gases im Bereich des Entladungsspalts. Die Dekontamination kann durch UVC-Strahlen erfolgen, die durch das Plasma abgestrahlt werden kann und/oder von Ozon, das sich im Entladungsspalt bilden kann, wenn es sich bei dem Gas um Luft handelt.

Durch die Erfindung ergibt sich der Vorteil, dass durch den Entladungsspalt eine Partikelfalle bereitgestellt ist, in welcher Partikel länger verbleiben als Moleküle des Gases selbst. Aufgrund des längeren Verbleibs der Partikel im Bereich des Entladungsspalts sind diese über einen längeren Zeitraum Plasmawirkungen wie Radikale und UVC-Strahlung ausgesetzt, wodurch eine Wahrscheinlichkeit einer Inaktivierung der Partikel zunimmt. Es kann sich beispielsweise bei einer UVC-Leistung von 100W/m2 und einer Verweilzeit der Partikel von nur 1s eine Dosisleistung von 100 J/m2 ergeben. Coronaviren sind ab 37 J/m2 schon zu 90% vollständig inaktiviert.

Die Erfindung umfasst auch Weiterbildungen, durch die sich weitere Vorteile ergeben.

Eine Weiterbildung der Erfindung sieht vor, dass jede der Kompositelektroden eine Kammstruktur aufweist, welche Elektrodenfinger aufweist. Mit anderen Worten umfassen die Kompositelektroden eine jeweilige Kammstruktur aus Elektrodenfingern. Die Kammstruktur kann eine Anordnung von parallel zueinander ausgerichteten Elektrodenfingern sein, welche durch einen vorgegebenen Abstand beabstandet nebeneinander angeordnet sein können. Die Kammstrukturen der Kompositelektroden der Elektrodeneinrichtung sind ineinandergreifend und durch den Entladungsspalt getrennt zueinander angeordnet. Mit anderen Worten sind die Kammstrukturen der Kompositelektroden der Elektrodeneinrichtung derart zueinander angeordnet, dass zwischen zwei Elektrodenfingern einer der Kompositelektroden der Elektrodenfinger der anderen der Kompositelektroden angeordnet ist. Die Abstände zwischen den Elektrodenfingern der Kammstruktur der jeweiligen Kompositelektrode sind dabei derart ausgewählt, dass der Abstand größer ist als eine Breite des Elektrodenfingers der anderen Elektrode. Die Elektrodenfinger der Kompositelektroden kontaktieren sich nicht, sondern sind durch den Entladungsspalt räumlich voneinander getrennt. Die Elektrodenbleche der Kammstruktur kann metallische oder allgemein elektrisch hochleitfähige Materialen aufweisen. Aufgrund der ineinandergreifenden Kammstrukturen der Kompositelektroden der Elektrodeneinrichtung weist der Entladungsspalt in der Elektrodeneinrichtung eine Mäanderstruktur auf. Durch die Weiterbildung ergibt sich der Vorteil, dass durch den meanderförmigen Verlauf des Entladungsspalts zum einen eine ausgeprägtere Wirkung des Entladungsspalts als Partikelfalle erreicht wird als bei anderen Verläufen zum anderen einer optimalen antisymmetrischen elektromagnetischen Potentialkontur mit perfekt linear polarisierten elektrischen Felder gefolgt wird.

Eine Weiterbildung der Erfindung sieht vor, dass das Elektrodenblech jeder der Kompositelektroden Aluminium und oder eine Aluminiumlegierung aufweist. Mit anderen Worten weist ein Material des Elektrodenblechs Aluminium oder die Aluminiumlegierung auf.

Eine weitere Lösung der Erfindung sieht vor, dass das Elektrodenblech jeder der Kompositelektroden rostfreien Stahl, bevorzugt Edelstahl aufweist. Mit anderen Worten weist das Elektrodenblech ein Material auf, das rostfreien Edelstahl umfasst. Es kann sich beispielsweise um Stahl handeln, der Chrom aufweist.

Eine Weiterbildung der Erfindung sieht vor, dass die dielektrische Beschichtung jeder der Kompositelektroden einen oder mehrere Polymere aufweist. Mit anderen Worten weist die dielektrische Beschichtung ein Material auf, das einen oder mehrere Polymere aufweist. Es kann sich dabei insbesondere elektrisch isolierende Polymerwerkstoffe handeln.

Eine Weiterbildung der Erfindung sieht vor, dass die dielektrische Beschichtung jeder der Kompositelektroden einen oder mehrere Fluorkunststoffe aufweist. Mit anderen Worten weist die dielektrische Beschichtung ein Material auf, das Fluorpolymere umfasst. Dadurch ergibt sich der Vorteil, dass die dielektrische Beschichtung Polymere mit einem relativ hohen Isolationsvermögen aufweist.

Eine Weiterbildung der Erfindung sieht vor, dass die dielektrische Beschichtung jeder der Kompositelektroden Polyvinylidenfluorid (PVDF) aufweist. Mit anderen Worten weist die dielektrische Beschichtung ein Material auf, das Polyvinylidenfluorid (PVDF) umfasst.

Eine Weiterbildung der Erfindung sieht vor, dass die dielektrische Beschichtung jeder der Kompositelektroden Polytetrafluorethylen (PTFE) aufweist. Mit anderen Worten weist die dielektrische Beschichtung ein Material auf, das Polytetrafluorethylen (PTFE) umfasst. Die Verwendung von Polytetrafluorethylen (PTFE) weist den Vorteil auf, dass es sich um einen Stoff mit einer relativ hohen Korrosions- und Temperaturbeständigkeit handelt.

Eine Weiterbildung der Erfindung sieht vor, dass die dielektrische Beschichtung jeder der Kompositelektroden Graphitfluorid aufweist. Mit anderen Worten weist die dielektrische Beschichtung ein Material auf, das Graphitfluorid umfasst.

Eine Weiterbildung der Erfindung sieht vor, dass dielektrische Beschichtung jeder der Kompositelektroden eine oder mehrere Keramiken aufweist. Mit anderen Worten weist die dielektrische Beschichtung ein Material auf, das eine oder mehrere Keramiken umfasst. Es kann sich insbesondere um Keramiken der Gruppe der Elektrokeramiken handeln. Mögliche Keramiken können beispielsweise Titanate, insbesondere Bariumtitanat-Keramiken und/oder Bleititanatzirkonat-Keramiken umfassen. Die Verwendung von Keramiken weist den Vorteil auf, dass es sich um Stoffe einer relativ hohen Durchschlagsfestigkeit handelt.

Eine Weiterbildung der Erfindung sieht vor, dass die dielektrische Beschichtung jeder der Kompositelektroden Bariumtitanat aufweist. Mit anderen Worten weist die dielektrische Beschichtung ein Material auf, das Bariumtitanat umfasst. Die Verwendung von Bariumtitanat weist den Vorteil auf, dass es sich um einen Stoff einer relativ hohen Dielektrizitätskonstante handelt.

Eine Weiterbildung der Erfindung sieht vor, dass die dielektrische Beschichtung jeder der Kompositelektroden Kaoloinit aufweist. Mit anderen Worten weist die dielektrische Beschichtung ein Material auf, das Kaolinit umfasst. Die Verwendung von Kaolinit weist den Vorteil auf, dass es sich um einen Stoff einer relativ niedrigen Dielektrizitätskonstante handelt.

Eine Weiterbildung der Erfindung sieht vor, dass die dielektrische Beschichtung jeder der Kompositelektroden einen Blend, umfassend vorgegebene Anteile von Kaoloinit, Aluminiumoxid, Titanoxid, Chromoxid, Bariumtitanat und/oder anderen keramischen Pulvern aufweist. Mit anderen Worten weist die dielektrische Beschichtung jeder der Kompositelektroden spezifische Anteile von Kaoloinit, Aluminiumoxid, Titanoxid, Chromoxid oder Bariumtitanat oder andere keramische Pulver aufweisen. Mit anderen Worten weist die dielektrische Beschichtung ein Material auf, das einen keramischen Blend umfasst. Die Verwendung des Blends weist den Vorteil auf, dass es sich um einen Stoff einer spezifisch optimal angepassten Dielektrizitätskonstante handelt. Ein Blend kann auch als Gemisch bezeichnet sein.

Eine Weiterbildung der Erfindung sieht vor, dass die Plasma-Filtereinrichtung zumindest zwei der Elektrodeneinrichtungen aufweist. Mit anderen Worten weist die Plasma-Filtereinrichtung zwei oder mehr als zwei der Elektrodeneinrichtungen auf. Die Plasma-Filtereinrichtung weist eine Halteeinrichtung als Gehäuse auf, welche dazu eingerichtet ist, die zumindest zwei der Elektrodeneinrichtungen in einer vorbestimmten Elektrodenanordnung anzuordnen, wobei die zumindest zwei der Elektrodeneinrichtungen in der vorbestimmten Elektrodenanordnung parallel zueinander ausgerichtet und entlang der Hauptströmungsrichtung hintereinander angeordnet sind. In der vorbestimmten Elektrodenanordnung sind benachbarte Elektrodeneinrichtungen durch einen vorbestimmten Abstand paarweise voneinander getrennt angeordnet. Mit anderen Worten ist die Plasma-Filtereinrichtung mittels der Halteeinrichtung dazu eingerichtet, die Anordnung der Elektrodeneinrichtungen in der vorbestimmten Elektrodenanordnung bereitzustellen. Es ist vorgesehen, dass die Elektrodeneinrichtungen durch die Halteeinrichtung parallel zueinander ausgerichtet sind. Es ist zudem vorgesehen, dass die Elektrodeneinrichtungen durch die Halteeinrichtung derart angeordnet sind, dass diese entlang der Hauptströmungsrichtung hintereinander angeordnet sind. Die Elektrodeneinrichtungen können beispielsweise entlang der Hauptströmungsrichtung zueinander verschoben sein. Die Halteeinrichtung ist dazu eingerichtet die zumindest zwei Elektrodeneinrichtungen der Art in der Elektrodenanordnung anzuordnen, dass diese paarweise durch einen vorbestimmten Abstand voneinander getrennt sind. Die Halteeinrichtung kann beispielsweise einen Abtrennrahmen aufweisen, welcher zwischen zwei jeweiligen der Elektrodeneinrichtung angeordnet ist und den vorbestimmten Abstand der Elektrodeneinrichtungen vorgeben kann. Durch die Weiterbildung ergibt sich der Vorteil, dass das Gas entlang der Hauptströmungsrichtung durch mehrere hintereinander angeordnete Elektrodeneinrichtungen geführt werden kann, wodurch mehrere Deaktivierungsschritte während des Durchströmens der Filtereinrichtung mittels Gas erfolgen.

Eine Weiterbildung der Erfindung sieht vor, dass benachbarte Elektrodeneinrichtungen der zumindest zwei Elektrodeneinrichtungen um 90° um die Hauptströmungsrichtung zueinander rotiert ausgerichtet sind. Mit anderen Worten ist es vorgesehen, dass hintereinander angeordnete Elektrodeneinrichtungen zueinander um 90° verdreht sind. Es kann beispielsweise vorgesehen sein, dass die Elektrodeneinrichtungen ein gleiches, durch den Entladungsspalt vorgegebenes Muster in der Hauptflächenebene aufweisen, wobei das Muster zwischen benachbarten Elektrodeneinrichtungen um 90° zueinander gedreht ist. Dadurch können die Muster der Elektrodeneinrichtungen entlang der Hauptströmungsrichtung eine Kreuzstruktur bilden. Durch die Weiterbildung ergibt sich der Vorteil, dass eine Verweilzeit der Aerosole und bevorzugt der Schwebeaerosole in benachbarten Elektrodeneinrichtungen erhöht werden kann.

Eine Weiterbildung der Erfindung sieht vor, dass die Plasma-Filtereinrichtung zumindest einen Staubfilter aufweist. Der zumindest eine Staubfilter ist in einer Hauptströmungsrichtung vor der Elektrodeneinrichtung angeordnet. Mit anderen Worten ist die Plasma-Filtereinrichtung dazu eingerichtet, das Gas entlang der Hauptströmungsrichtung zuerst durch den Staubfilter zu führen, bevor das Gas durch die Elektrodeneinrichtung geführt wird. Bei dem Staubfilter kann es sich beispielsweise um einen mechanischen Staubfilter handeln, der ein Gewebe umfasst, durch welches das Gas zur Filterung geführt wird. Es kann sich auch um einen elektrostatischen Staubfilter handeln. Durch die Weiterbildung ergibt sich der Vorteil, dass Schmutzpartikel oder größere Partikel durch den Staubfilter aufgefangen werden können, sodass ein Verstopfen der Elektrodeneinrichtung aufgrund von Staub oder Verunreinigungen verzögert oder verhindert werden kann.

Eine Weiterbildung der Erfindung sieht vor, dass die Plasma-Filtereinrichtung zumindest einen Aktivkohlefilter aufweist. Es ist vorgesehen, dass der zumindest eine Aktivkohlefilter in einer Hauptströmungsrichtung hinter der Elektrodeneinrichtung angeordnet ist. Mit anderen Worten ist die Plasma-Filtereinrichtung dazu eingerichtet, das Gas entlang der Hauptströmungsrichtung nach dem Durchströmen der Elektrodeneinrichtung durch den zumindest einen Aktivkohlefilter zu führen. Durch die Weiterbildung ergibt sich der Vorteil, dass durch den Aktivkohlefilter bestimmte, unerwünschte kurzlebige Radikale (plasmaspezifische transient ladungstragende Ionen/Moleküle/Atome), die sich in dem Plasma gebildet haben können, durch Neutral-Rekombination katalytisch herausgefiltert werden können. Es kann beispielsweise aufgrund der Plasmabildung in dem Entladungsspalt zu einer Bildung von Ozon kommen. Eine Ausgabe von Ozon ist jedoch in bestimmten Situationen unerwünscht. Aufgrund der Bereitstellung des Aktivkohlefilters wird das entstandene Ozon rekombiniert, sodass der Ozonanteil des die Plasma-Filtereinrichtung austretenden Gases katalytisch auf ein für Reinluft tolerables Maß verringert werden kann.

Ein zweiter Aspekt der Erfindung betrifft eine Elektrodeneinrichtung für eine Plasma-Filtereinrichtung. Die Elektrodeneinrichtung ist dazu vorgesehen in einer Plasma-Filtereinrichtung zur Dekontamination eines Gases angeordnet zu werden.

Es ist vorgesehen, dass die Elektrodeneinrichtung eine erste plan ausgebildete Kompositelektrode und eine zweite plan ausgebildete Kompositelektrode aufweist. Mit anderen Worten umfasst die Elektrodeneinrichtung die erste Kompositelektrode und die zweite Kompositelektrode. Die Kompositelektroden der Elektrodeneinrichtung können insbesondere als Flächenelemente eingerichtet sein. Es ist vorgesehen, dass die Kompositelektroden der Elektrodeneinrichtung coplanar zueinander in einer Hauptflächenebene der Elektrodeneinrichtung angeordnet und durch einen Entladungsspalt räumlich voneinander getrennt sind. Mit anderen Worten befinden sich die Kompositelektroden der Elektrodeneinrichtung in der Hauptflächenebene der Elektrodeneinrichtung. Zwischen den Kompositelektroden der Elektrodeneinrichtung befindet sich ein Entladungsspalt. Bei dem Entladungsspalt handelt es sich um einen durch die Kompositelektroden gebildeten Spalt der Elektrodeneinrichtung zum Durchleiten des zu filternden Gases. Es ist vorgesehen, dass jede der Kompositelektroden ein jeweiliges Elektrodenblech aufweist, das zumindest an einer Grenzfläche des jeweiligen Elektrodenblechs zu dem Entladungsspalt eine jeweilige dielektrische Beschichtung aufweist. Mit anderen Worten sind die Elektroden als Komposite bereitgestellt, welche das jeweilige Elektrodenblech und eine auf dem jeweiligen Elektrodenblech befindliche dielektrische Beschichtung umfassen. Die dielektrische Beschichtung ist zumindest an einer von der jeweiligen Kompositelektrode an dem Entladungsspalt angrenzende Grenzfläche auf dem Elektrodenblech aufgetragen.

Weitere Ausführungsformen der erfindungsgemäßen Elektrodeneinrichtung folgen aus den verschiedenen Ausführungsformen der erfindungsgemäßen Plasma-Filtereinrichtung.

Ein dritter Aspekt der Erfindung betrifft ein Verfahren zum Betreiben einer Plasma-Filtereinrichtung.

Die Plasma-Filtereinrichtung weist zumindest eine Elektrodeneinrichtung auf, wobei die Elektrodeneinrichtung eine erste plan ausgebildete Kompositelektrode und eine zweite plan ausgebildete Kompositelektrode aufweist. Mit anderen Worten umfasst die Elektrodeneinrichtung die erste Kompositelektrode und die zweite Kompositelektrode. Die Kompositelektroden der Elektrodeneinrichtung können insbesondere als Flächenelemente eingerichtet sein. Es ist vorgesehen, dass die Kompositelektroden der Elektrodeneinrichtung coplanar zueinander in einer Hauptflächenebene der Elektrodeneinrichtung angeordnet und durch einen Entladungsspalt räumlich voneinander getrennt sind. Mit anderen Worten befinden sich die Kompositelektroden der Elektrodeneinrichtung in der Hauptflächenebene der Elektrodeneinrichtung. Zwischen den Kompositelektroden der Elektrodeneinrichtung befindet sich ein Entladungsspalt. Bei dem Entladungsspalt handelt es sich um einen durch die Kompositelektroden gebildeten Spalt der Elektrodeneinrichtung zum Durchleiten des zu filternden Gases. Es ist vorgesehen, dass jede der Kompositelektroden ein jeweiliges Elektrodenblech aufweist, das zumindest an einer Grenzfläche des jeweiligen Elektrodenblechs zu dem Entladungsspalt eine jeweilige dielektrische Beschichtung aufweist. Mit anderen Worten sind die Elektroden als Komposite bereitgestellt, welche das jeweilige Elektrodenblech und eine auf dem jeweiligen Elektrodenblech befindliche dielektrische Beschichtung umfassen. Die dielektrische Beschichtung ist zumindest an einer von der jeweiligen Kompositelektrode an dem Entladungsspalt angrenzende Grenzfläche auf dem Elektrodenblech aufgetragen.

In dem Verfahren ist vorgesehen, dass durch eine Spannungsquelle der Plasma-Filtereinrichtung eine Wechselspannung, vorzugsweise im erhöhten Wechselspannungsbereich (100-1000 Hz) oder im unteren Hochfrequenzbereich (Kilohertz) wie auch im Hoch- und Höchstfrequenzbereich (1GHz-100 GHz) an der Elektrodeneinrichtung bereitstellt wird, wodurch ein Plasma aufgrund einer dielektrischen Barriereentladung in dem Entladungsspalt hervorgerufen gebildet wird. Durch die Plasma-Filtereinrichtung wird ein Gas entlang einer Hauptströmungsrichtung, welche parallel zu einer Normalen der Hauptflächenebene der Elektrodeneinrichtung ausgerichtet ist durch den Entladungsspalt geführt. Aufgrund des Plasmas kommt es zu einer Dekontamination des Gases wobei beispielsweise Keime abgetötet werden.

Weitere Ausführungsformen des erfindungsgemäßen Verfahrens folgen aus den verschiedenen Ausführungsformen der erfindungsgemäßen Plasma-Filtereinrichtung sowie der erfindungsgemäßen Elektrodeneinrichtung.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein. Es können insbesondere auch Ausführungen und Merkmalskombinationen von der Erfindung umfasst sein, die nicht alle Merkmale eines ursprünglich formulierten Anspruchs aufweisen. Es können darüber hinaus Ausführungen und Merkmalskombinationen von der Erfindung umfasst, die über die in den Rückbezügen der Ansprüche dargelegten Merkmalskombinationen hinausgehen oder von diesen abweichen.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt. In den Figuren zeigen:
- FIG 1: eine schematische Darstellung einer Elektrodeneinrichtung für eine Plasma-Filtereinrichtung;
- FIG 2: eine schematische Darstellung einer Elektrodeneinrichtung;
- FIG 3: eine schematische Darstellung einer Funktionsweise der elektrischen Elektrodeneinrichtungen;
- FIG 4: eine schematische Darstellung einer Plasma-Filtereinrichtung;
- FIG 5: eine schematische Darstellung einer Plasma-Filtereinrichtung;
- FIG 6: eine schematische Darstellung eine schematische Darstellung einer Simulation eines Luftvolumenstroms durch die Plasma-Filtereinrichtung;
- FIG 7: eine schematische Darstellung einer Aerosol-Transmission durch eine Elektrodeneinrichtung;
- FIG 8: eine schematische Darstellung einer Aerosol-Transmission durch zwei Elektrodeneinrichtungen; und
- FIG 9: eine schematische Darstellung von Anordnungsmöglichkeiten der Elektrodeneinrichtungen.

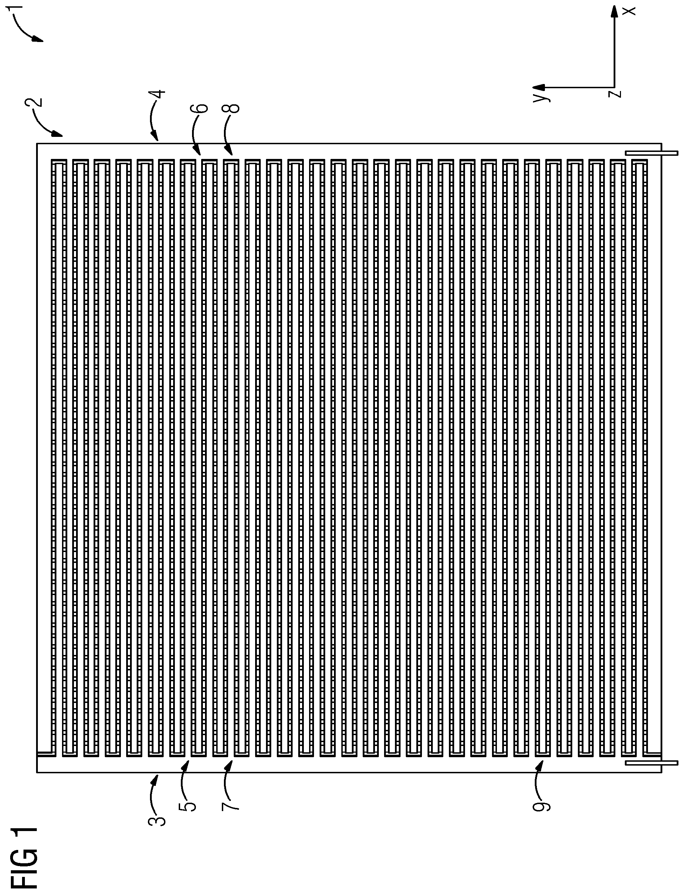

FIG 1 zeigt eine schematische Darstellung einer Elektrodeneinrichtung für eine Plasma-Filtereinrichtung.

Die Elektrodeneinrichtung 2 der Plasma-Filtereinrichtung 1 kann eine erste Kompositelektrode 3 und eine zweite Kompositelektrode 4 aufweisen. Die erste Kompositelektrode 3 und die zweite Kompositelektrode 4 können in einer Hauptflächenebene 13 der Elektrodeneinrichtung 2 liegen und plan sein. Die erste Kompositelektrode 3 und die zweite Kompositelektrode 4 können beispielsweise coplanar zueinander angeordnet sein. Die Kompositelektroden 3, 4 können aus einem Ursprungsblech gefertigt worden sein, welches durch eine Bereitstellung eines Entladungsspaltes 9 in dem Ursprungsblech in ein erstes Elektrodenblech 5 und ein zweites Elektrodenblech 6 aufgeteilt worden sein kann, wobei das erste Elektrodenblech 5 und das zweite Elektrodenblech 6 durch den Entladungsspalt 9 voneinander getrennt sein können. Die beiden Kompositelektroden 3, 4 können ein jeweiliges Elektrodenblech 5, 6 aufweisen, welches mit einer jeweiligen dielektrischen Beschichtung 7, 8 beschichtet sein kann, wodurch es sich bei den jeweiligen Elektroden um Komposite handeln kann. Das Elektrodenblech 6, 6 der jeweiligen Kompositelektrode 3, 4 kann beispielsweise Aluminium, eine Aluminiumlegierung und/oder rostfreien Stahl als Material aufweisen. Die dielektrische Beschichtung 7, 8 der jeweiligen Kompositelektrode 3, 4 kann zumindest an einer Grenzfläche der jeweiligen Kompositelektrode 3, 4 zu dem Entladungsspalt 9 auf dem jeweiligen Elektrodenblech 5, 6 aufgetragen sein. Die Aufgabe der dielektrischen Beschichtung 7, 8 kann darin bestehen, eine dielektrische Entladung in dem Entladungsspalt 9 zu ermöglichen, wenn eine entsprechend parameterisierte elektrische Wechselspannung an der Elektrodeneinrichtung 2 angelegt ist. Die dielektrische Beschichtung 7, 8 kann einen oder mehrere Polymere als Material aufweisen. Die möglichen Polymere können beispielsweise Fluorkunststoffe, insbesondere Polyvinylidendifluorid und/oder Polytetrafluorethylen aufweisen. Dem zumindest einen Polymer kann auch Graphitfluorid beigemischt sein. Die dielektrische Beschichtung 7, 8 kann auch eine oder mehrere Keramiken als Material, insbesondere Bariumtitanat aufweisen.

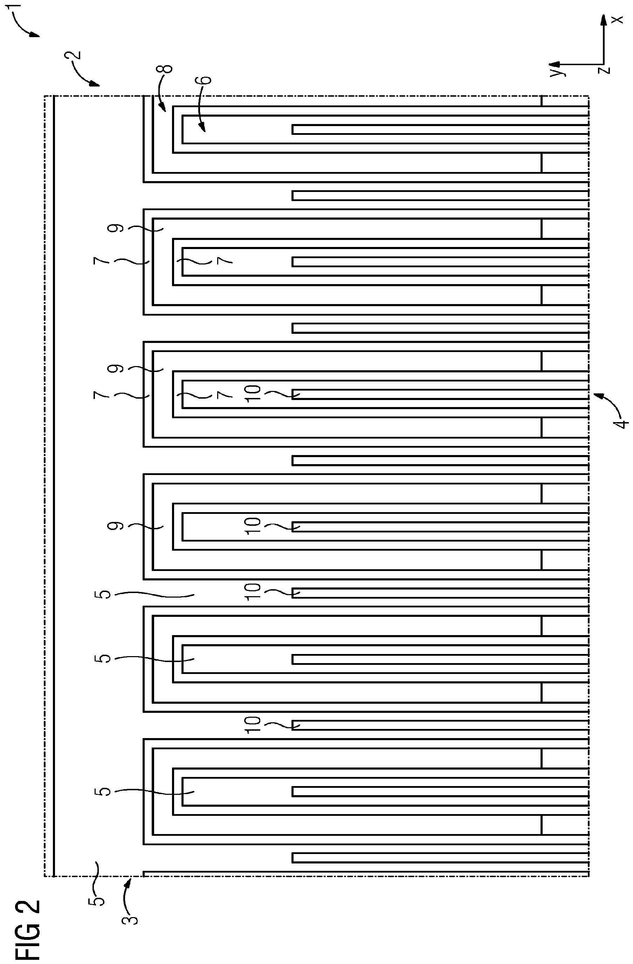

FIG 2 zeigt eine schematische Darstellung einer Elektrodeneinrichtung.

FIG 2 zeigt Kammstrukturen der Kompositelektroden 3, 4 der Elektrodeneinrichtung 2. Die erste Kompositelektrode 3 der Elektrodeneinrichtung 2 kann eine Kammstruktur aufweisen, welche Elektrodenfinger umfassen kann, welche parallel und beabstandet 15 zueinander angeordnet sein können. Entsprechend kann auch die zweite Kompositelektrode 4 der Elektrodeneinrichtung 2 eine Kammstruktur mit Elektrodenfinger aufweisen, welche in Zwischenräume zwischen den Kompositelektroden 3, 4 eingreifen können. Die Elektrodenfinger der jeweiligen Kompositelektroden 3, 4 können durch den Entladungsspalt 9 voneinander beabstandet 15 sein. Die Oberflächen der Elektrodenbleche 5, 6 können die dielektrischen Beschichtungen 7, 8 zumindest im Bereich des Entladungsspalts 9 aufweisen. Die Kammstrukturen können auch dielektrische Lücken 10 aus Dielektrika umfassen, welche auf den Elektrodenblechen 5, 6 entlang jeweiliger Mittellinien der Elektrodenfinger aufgetragen sein können.

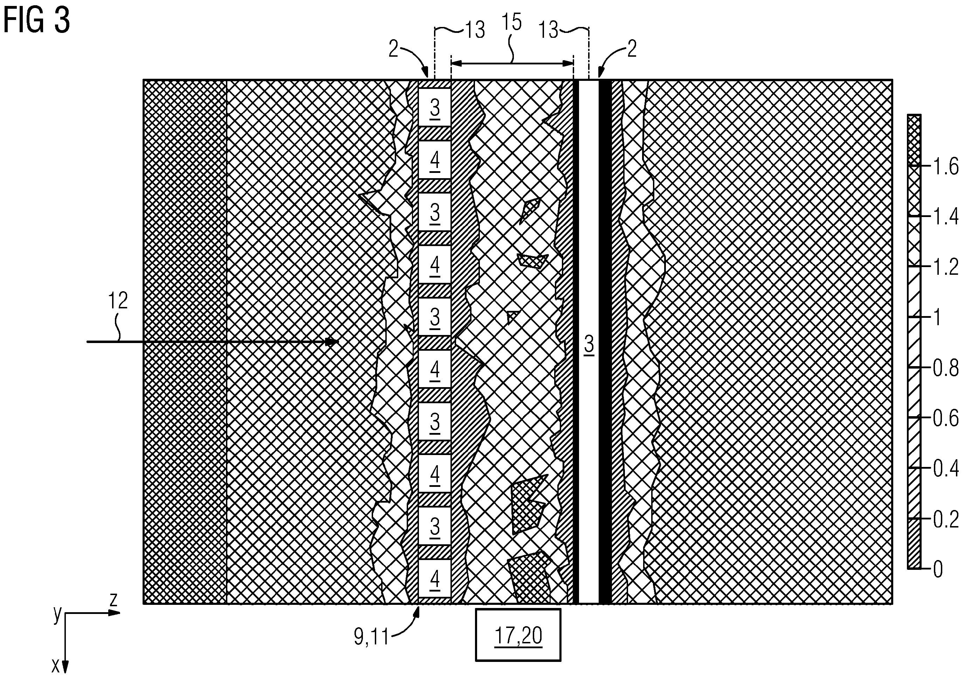

FIG 3 zeigt eine schematische Darstellung einer Funktionsweise der elektrischen Elektrodeneinrichtungen.

Gezeigt sind zwei der Elektrodeneinrichtungen 2, welche bezüglich einer Hauptströmungsrichtung 12 des Gases durch die Plasma-Filtereinrichtung 1 hintereinander angeordnet sein können. Die Plasma-Filtereinrichtung 1 kann dazu vorgesehen sein, das zu dekontaminierende Gas entlang der Hauptströmungsrichtung 12 des Gases durch die erste Elektrodeneinrichtung 2 und die zweite Elektrodeneinrichtung 2 zu leiten, wobei die Hauptströmungsrichtung 12 parallel zu einer Normalen der Hauptflächenebenen 13 der Elektrodeneinrichtungen 2 ausgerichtet sein kann. Gezeigt ist eine Verteilung von Geschwindigkeitsbeträgen in m/s von Partikeln, welche sich in dem durchgeleiteten Gas befinden können. Aufgrund der senkrechten Ausrichtung der Hauptströmungsrichtung 12 bezüglich der Elektrodeneinrichtung 2 wird das Gas durch die Entladungsspalten 9 der Elektrodeneinrichtungen 2 geführt. Durch eine nicht gezeigte Spannungsquelle 22 kann eine Wechselspannung an den Elektrodeneinrichtungen 2 bereitgestellt sein, welche zu einer Ausbildung eines Plasmas 11 in den Entladungsspalten 9 aufgrund einer dielektrischen Entladung führen kann. Das Plasma 11 kann UV-Strahlung abstrahlen, welche Aerosole im Gas deaktivieren kann. Die Deaktivierung kann ein Hervorrufen einer bestimmten chemischen Reaktion in einem Aerosol umfassen, wodurch beispielsweise Keime abgetötet werden können. Die Elektrodeneinrichtungen 2 können durch eine Halteeinrichtung 20 in einer vorgegebenen Elektrodenanordnung angeordnet sein und durch einen Abstand 15 voneinander getrennt sein. In der Kartierung des Geschwindigkeitsbetrags ist zu erkennen, dass die Geschwindigkeit um die beiden Elektrodeneinrichtungen 2 ein Minimum aufweist. Dies ist darauf zurückzuführen, dass die in der FIG 2 beschriebene Mäanderform des Entladungsspalts 9 dazu führt, dass die Entladungsspalte 9 als Partikelfalle fungiert, wodurch sich Partikel des Gases über einen längeren Zeitraum in dem jeweiligen Entladungsspalt 9 aufhalten. Aufgrund der größeren Aufenthaltszeit der Partikel in dem Entladungsspalt 9 ist ein Partikel über einen längeren Zeitraum dem Plasma 11 und somit der ultravioletten Strahlung ausgesetzt.

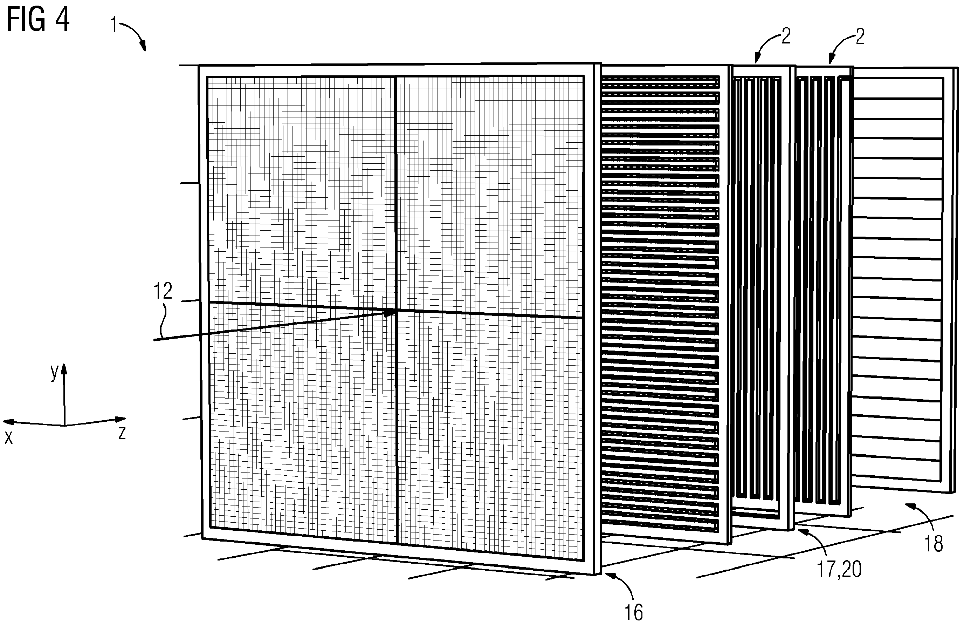

FIG 4 zeigt eine schematische Darstellung einer Plasma-Filtereinrichtung.

Die Plasma-Filtereinrichtung 1 kann einen Staubfilter 16 aufweisen, welcher bezüglich einer Hauptströmungsrichtung 12 vor den Elektrodeneinrichtungen 2 angeordnet sein kann. Der Staubfilter 16 kann dazu vorgesehen sein, Staubpartikel oder größere Aerosole aus dem, durch die Plasma-Filtereinrichtung 1 geführtem Gas vor dem Durchlaufen der Elektrodeneinrichtung 2 herauszufiltern, um einen Verstopfen und/oder eine Verunreinigung der Elektrodeneinrichtung 2 zu verhindern oder zu verlangsamen. Hinter dem Staubfilter 16 kann eine erste Elektrodeneinrichtung 2 angeordnet sein. Die Elektrodeneinrichtung 2 kann die beschriebene Meanderstruktur aufweisen, wobei die Elektrodenfinger entlang der X-Richtung ausgerichtet sein können. Hinter der ersten Elektrodeneinrichtung 2 kann die weitere Elektrodeneinrichtung 2 angeordnet sein, welche durch einen Abtrennrahmen 17 von der ersten Elektrodeneinrichtung 2 in einem vorgegebenen Abstand 15 angeordnet sein kann. Die zweite Elektrodeneinrichtung 2 kann um 90° bezüglich der entlang einer Z-Richtung verlaufenden Normalen gedreht sein. In dieser Ausrichtung können die Elektrodenfinger beispielsweise parallel zur Y-Richtung ausgerichtet sein. Durch die gedrehte Ausrichtung der nachfolgenden Elektrodeneinrichtung 2 gegenüber der ersten Elektrodeneinrichtung 2 kann eine Aufenthaltsdauer der Partikel in Entladungsspalten 9 der Plasma-Filtereinrichtung 1 gegenüber Plasma-Filtereinrichtungen 1 mit zueinander unverdrehten Elektrodeneinrichtungen 2 verlängert sein. Hinter der zweiten Elektrodeneinrichtung 2 kann ein Aktivkohlefilter 18 angeordnet sein, welcher durch das Gas durchströmt wird. Der Aktivkohlefilter 18 kann dazu vorgesehen sein, Ozonmoleküle, welche sich im Plasma 11 bilden können, aus dem Gas herauszufiltern.

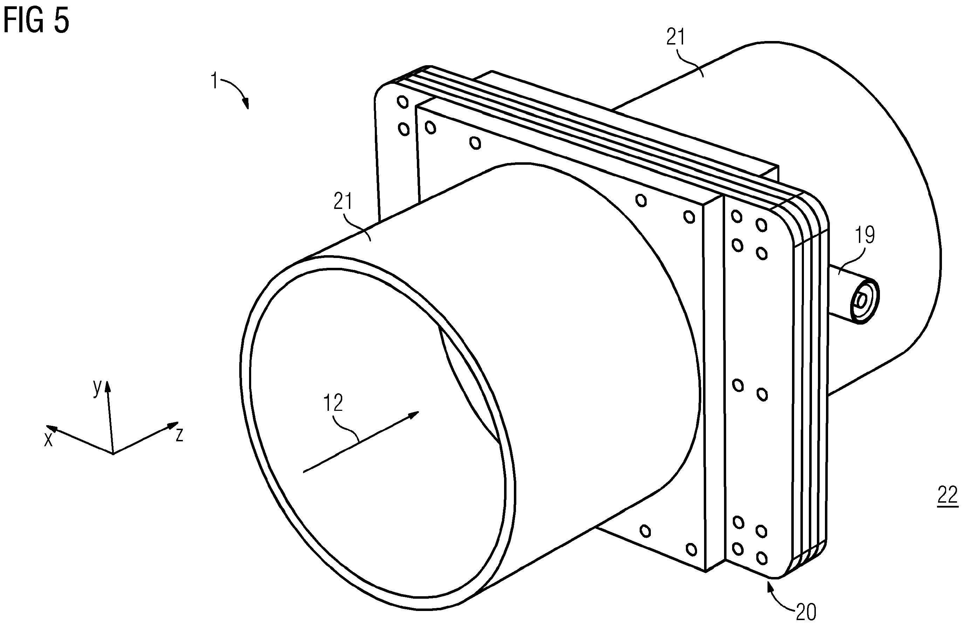

FIG 5 zeigt eine schematische Darstellung einer Filtereinrichtung.

Die Plasma-Filtereinrichtung 1 kann eine Halteeinrichtung 20 aufweisen, welche dazu vorgesehen sein kann, die Elektrodeneinrichtungen 2, den Staubfilter 16 und den Aktivkohlefilter 18 in vorgegebenen Lagen anzuordnen. An der Halteinrichtung kann ein Einspeisepunkt 19 angeordnet sein, welcher dazu vorgesehen sein kann, die Elektrodeneinrichtungen 2 mit der Wechselspannung der Spannungsquelle 22 zu versorgen. Um ein Durchleiten des Gases entlang der Hauptströmungsrichtung 12 zu erreichen, können an der Plasma-Filtereinrichtung 1 ein oder zwei Röhren 21 oder allgemein Führungselemente angeordnet sein, welche über einen Flansch an der Halteeinrichtung 20 angeordnet sein können.

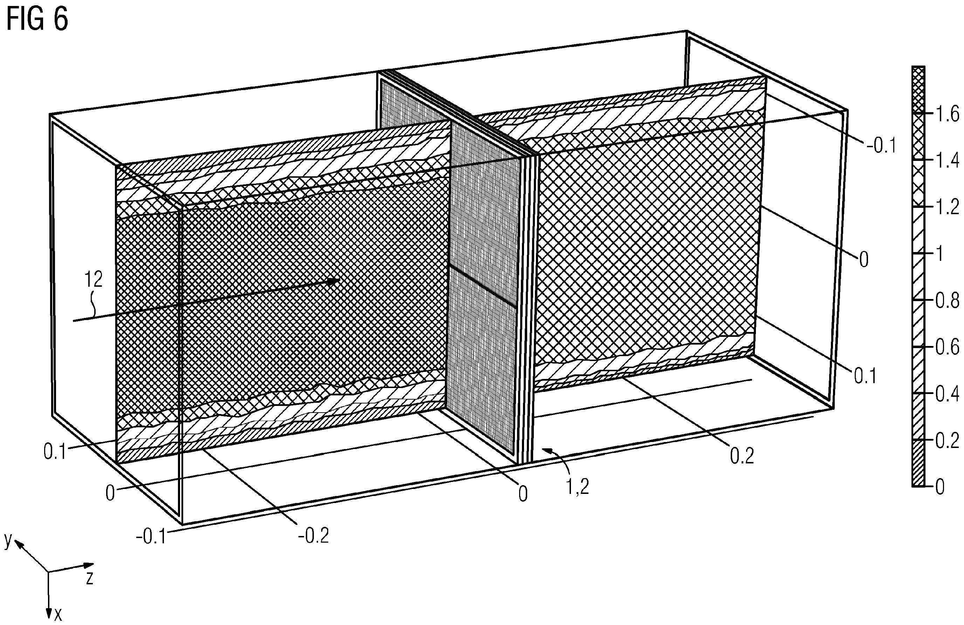

FIG 6 zeigt eine schematische Darstellung einer Simulation eines Luftvolumenstroms durch eine Plasma-Filtereinrichtung.

Gezeigt ist eine Simulation des Luftvolumenstroms durch die vorliegende Plasma-Filtereinheit in m/s.

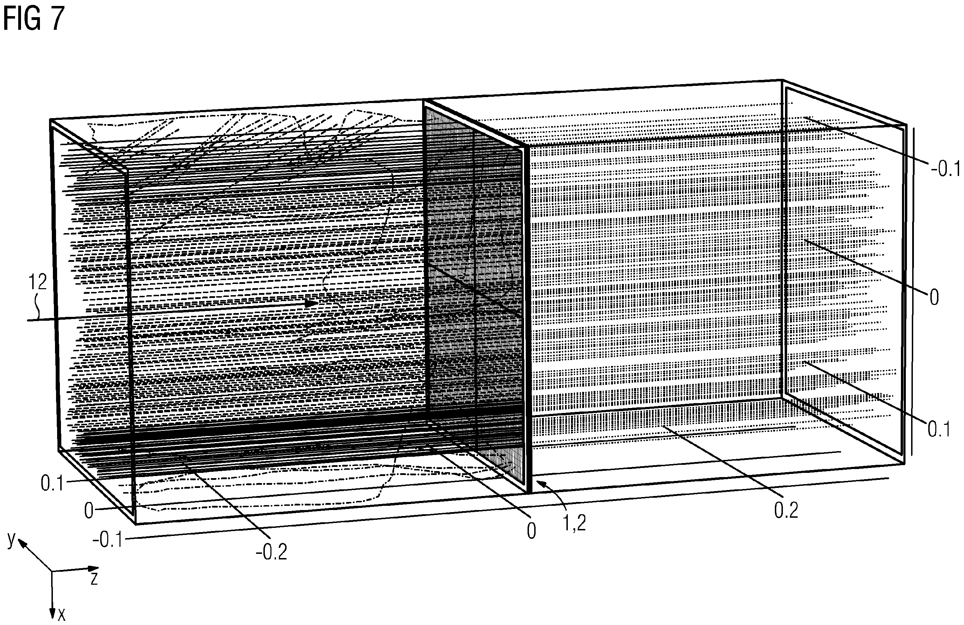

FIG 7 zeigt eine schematische Darstellung einer Aerosol-Transmission durch eine Elektrodeneinrichtung.

Die Figur FIG 7 zeigt eine schematische Darstellung einer Aerosol-Transmission von Aerosolen einer Größe von 0,3 µm bei einer Zeit von 600 Sekunden durch eine Elektrodeneinrichtung 2. Die Simulation zeigt, dass die Aerosole der Größe von 0,3 µm eine relativ hohe Verweilzeit in den meanderförmigen Entladungspalten 9 aufweisen. Dargestellt ist die Geschwindigkeit der Aerosole in m/s.

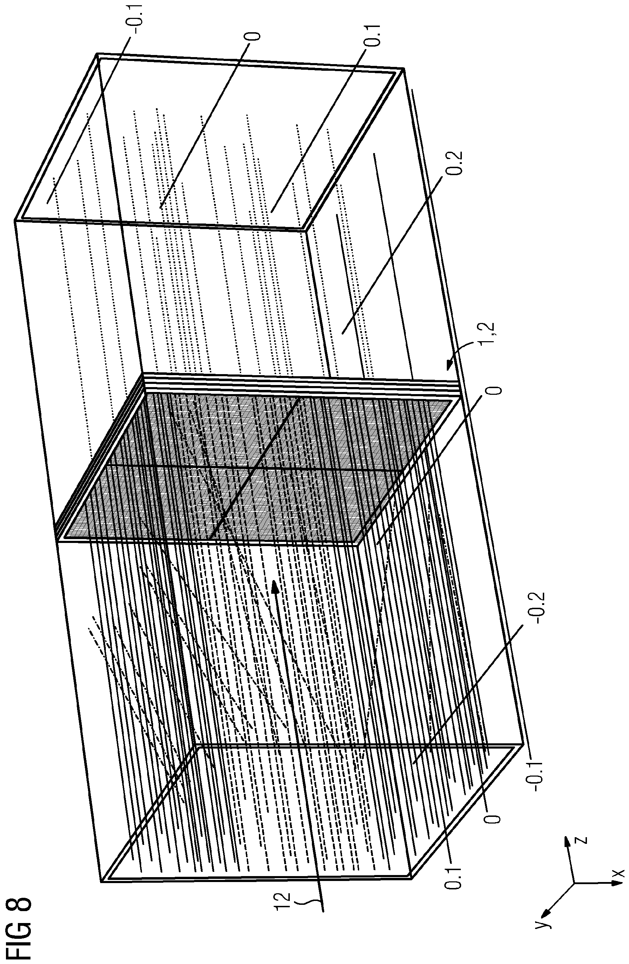

FIG 8 zeigt eine schematische Darstellung einer Aerosol-Transmission durch zwei Elektrodeneinrichtungen.

Die Figur zeigt eine schematische Darstellung einer Aerosol-Transmission von Aerosolen einer Größe von 0,3 µm bei 600 Sekunden durch zwei der Elektrodeneinrichtungen 2. Dargestellt ist die Geschwindigkeit der Aerosole in m/s. Die zwei Elektrodeneinrichtungen 2 sind hintereinander angeordnet und um 90° zueinander verdreht. Zu erkennen ist, dass der Aufenthalt der Partikel in der Plasma-Filtereinrichtung 1, welche zwei Elektrodeneinrichtungen 2 umfasst gegenüber dem Aufenthalt in der Plasma-Filtereinrichtung 1, welche eine Elektrodeneinrichtung 2 aufweist, verlängert ist. Dies ist im Vergleich der FIG 7 mit der FIG 8 zu erkennen.

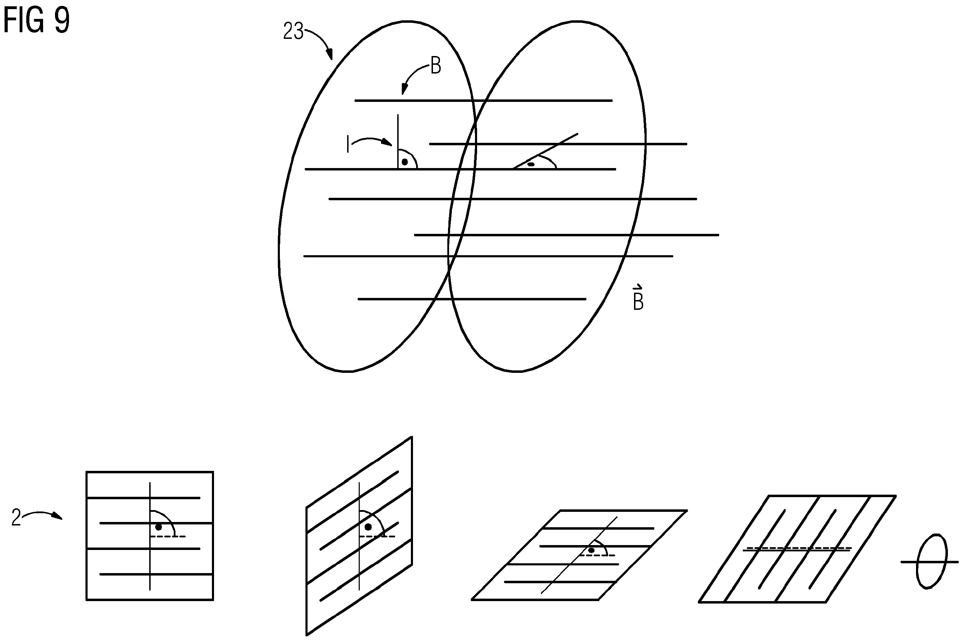

FIG 9 zeigt eine schematische Darstellung von Anordnungsmöglichkeiten der Elektrodeneinrichtungen 2.

Die Elektrodeneinrichtungen 2 zeichnen sich durch ihre bevorzugte lineare Anisotrope Richtung aus. FIG 9 zeigt die Möglichkeiten, die Elektrodeneinrichtungen 2 so auszurichten, dass eine vollständige Unterdrückung jeglicher Beeinflussung des Plasmas 11 entstehen kann. Gezeigt sind Helmholtz-Spulen 23 einer MRT-Vorrichtung mit der B-Feld-Orientierung B und Orientierungen von Elektrodeneinrichtungen 2 mit Plasmastromorientierungen I.

Technische Merkmale der Erfindung und dadurch erzielte Vorteile durch die Verwendung von Kompositelektroden 3, 4 mit definiertem Design und Anordnung. Die Anordnung wird im Nachfolgenden als Plasma-Filtereinrichtung 1 bezeichnet.

Die Erfindung umfasst zwei Kerngedanken. Der erste Kerngedanke betrifft eine Erzeugung eines Plasmas 11, um hohe Photonen-Dichten im UVC-Wellenlängenbereich zu erzeugen. Der zweite Kerngedanke betrifft eine Bereitstellung eines neuen Elektroden- und Filterdesigns, durch welches eine längere Verweilzeit von bestimmten Aerosol-Partikelfraktionen aus einem laminaren Gasvolumenstrom in der Plasma-Filtereinrichtung 1 erzwungen wird.

Nachfolgende Ausführungen sollen die Ausprägungen des Aufbaus der Plasma-Filtereinrichtung 1 zur Gasdekontamination detailliert beschreiben und die beiden Kerngedanken genauer erläutern.

Der erste Kerngedanke, betrifft die Erzeugung des Plasmas 11 über ein definiertes Elektroden-Design. Dieser Kerngedanke umfasst eine Kombination der zwei metallischen Elektrodenbleche 5, 6, durch welche eine Meanderstruktur der Elektrodeneinrichtung 2 bereitgestellt ist. Die Herstellung der zwei Elektrodenbleche 5, 6 der Elektrodeneinrichtung 2 kann mittels kommerzieller und kostengünstiger Herstellungsverfahren nach dem Stand der Technik, beispielsweise durch ein Stanzen und/oder Schneiden eines Ursprungsblechs erfolgen. Das Ursprungsblech kann dabei derart bearbeitet werden, dass der meanderförmige Verlauf des Entladungsspalts 9 in dem Ursprungsblech bereitgestellt wird. Durch den Entladungsspalt 9 kann das Ursprungsblech in die zwei Elektrodenbleche 5, 6 der Elektrodeneinrichtung 2 geteilt werden.

Auf den zwei Elektrodenblechen 5, 6 der Elektrodeneinrichtung 2 kann eine dielektrische Beschichtung 7, 8 aus einem Dielektrikum mit einer definierten Permittivität ε im Bereich von 5 - 50 aufgetragen werden, wodurch die Kompositelektroden 3, 4 bereitgestellt werden können. Die zwei Elektrodenbleche 5, 6 können vorzugsweise Al-, Al-Legierungen oder rostfreie Stähle umfassen.

Das Dielektrikum der dielektrischen Beschichtungen 7, 8 kann beispielsweise Polymere, zum Beispiel PVDF, PTFE und anderen fluorverwandten polarisierten Polymere mit einem sehr hohen Anteil an Graphitfluorid C-F mit einer Bindungsenergie von 489 kJ/mol aufweisen. Das Dielektrikum der dielektrischen Beschichtungen 7, 8 kann auch Keramiken, zum Beispiel Bariumtitanat mit einer Permittivität von 50 aufweisen.

Randbedingung der Simulation ist eine keramische Beschichtung 7, 8 mit einer erhöhten Dielektrizitätszahl von 12, um den charakteristischen Unterschied zu einer polymerbezogenen dielektrischen Beschichtung 7, 8 mit Epsilon = 7 zu zeigen. Die Dicke der polymerbezogenen dielektrischen Beschichtung 7, 8 ist aufgrund der technischen Anforderungen des Spritzgießprozesses zum Auftragen des zumindest einen Polymers gegenüber der Dicke einer keramischen dielektrischen Beschichtung 7, 8 höher. Erfahrungsgemäß die Werte der Dicke der polymerbezogenen dielektrischen Beschichtung 7, 8 kann beispielsweise 0,5 mm betragen. Die keramische dielektrische Beschichtung 7, 8 kann beispielsweise eine Dicke von 0,2 mm aufweisen. Die keramische dielektrische Beschichtung 7, 8 kann beispielsweise mittels einer chemischen Gasphasenabscheidung des Dielektrikums auf dem jeweiligen Elektrodenblech aufgetragen sein.

Die Analyse zeigt, dass eine Vergrößerung des Luftspalts innerhalb bestimmter Bereiche die abgeschiedene Leistung im Plasma 11 vorteilhaft erhöht.

Die Plasma-Filtereinrichtung 1 kann Elektrodeneinrichtungen 2 aus Kompositelektroden 3, 4, sowie flächige Staubfilter 16 und Aktivkohlefilter 18, wie in FIG gezeigt, aufweisen. Die Plasma-Filtereinrichtung 1 kann eine gegenüber bekannten Plasma-Filtereinrichtungen 1 sehr kleine Bauform aufweisen. Die Haltevorrichtung kann Rahmen und Einfassungen aufweisen, die polymere Materialien umfassen können.

Zur Dekontamination des Gases ist eine Erzeugung von Photonen im UVC-Wellenlängenbereich vorgesehen. Die Plasma-Filtereinrichtung 1 ist zur Erzeugung der Photonen im Luftspalt zwischen den Kompositelektroden 3, 4 der Elektrodeneinrichtung 2 mittels eines Plasmas 11 eingerichtet. Es ist vorgesehen, dass das Plasma 11 über eine dielektrische Barriereentladung in dem Entladungsspalt 9 erzeugt wird.

Das durch die Plasma-Filtereinrichtung 1 durchströmende Gas wird über das in dem Entladungsspalt 9 gezündete Plasma 11 elektrisch leitfähig. Durch den vorliegenden Ansatz entstehen durch hochenergetische Energieumwandlungen bei Elektronentemperaturen von 4-6 eV hohe Photonendichten >>10^15/s mit Wellenlängen im UVC Bereich, wie in FIG 2 zu erkennen.

Bei den vorliegenden UVC Wellenlängen entstehen durch das verwendete Dielektrikum und dem Elektrodenaufbau hohe Reflexionsraten von bis zu 97%, welche in bekannten aufbauten nach dem Stand der Technik nicht erreicht werden.

Im Gegensatz zur Verwendung marktverfügbarer UVC-Strahlungsquellen werden 1.000- bis 10.000-mal höhere Photonendichten erzeugt.

Um einen Vergleich einer 20W Situation zwischen UVC-Lampen und der Elektrode aus der vorliegenden Plasma-Filtereinheit abzuleiten, benötigt man 6 UV-C Lampen bei 20cm Länge, die einen luftdurchströmten Querschnitt durch ein UV-C-transparentes Lüftungsrohr 21 bestrahlen, das dabei auch durch ein Aluminiumrohr 21 mit hohem Reflexionsgrad für UV-Strahlung der ausgewählten Wellenlänge umschlossen ist.

Die im Luftstrom enthaltenen Aerosole durchschreiten den UVC-Wirkungsbereich über 20 cm in ca. 0,11s bei einer 100- bis 1000-mal geringeren Photonendichte gegenüber dem Meanderspalt der Elektrodeneinrichtung 2, wo die Aerosole durch den Partikelfalleneffekt im Minutenbereich verharren können. Die Inaktivierungsrate liegt daher um das Produkt Dichte x Zeit um viele Größenordnungen höher als bei konventionellen UV-C-Lampen. Durch Hintereinanderschalten von weiteren Elektrodeneinrichtungen 2 kann die Wirksamkeit der Plasma-Filtereinrichtung 1 noch weiter gesteigert werden.

Im Folgenden wird der genannte Partikelfalleneffekt zur Inaktivierung der Aerosole genauer beschrieben. Generell bestehen Aerosole aus festen oder flüssigen Partikeln unterschiedlicher Größe. Ein großer Teil sind solche im 100-pm-Bereich. Es kann aber auch von einem Anteil deutlich kleinerer Partikel, kleiner als 5 µm oder kleiner als 2,5 µm ausgegangen werden Quelle: DGUV Regel 102-001, Ausgabe September 2019, zu "Regeln für Sicherheit und Gesundheit bei Tätigkeiten mit Biostoffen im Unterricht" der Deutschen Gesetzlichen Unfallversicherung e.V. DGUV. Während Tröpfchen von 100 µm Durchmesser etwa 6 Sekunden benötigen, um aus einer Höhe von 2 m auf den Boden zu sinken, benötigen Tröpfchen von 10 µm Durchmesser 10 Minuten für die gleiche Entfernung, Tröpfchen von 1 µm benötigen 16,6 Stunden Quelle: Kappstein, Ines. Nosokomiale Infektionen: Prävention, Labordiagnostik, antimikrobielle Therapie ; 122 Tabellen. Deutschland, Thieme, 2009. Kleinere Partikel bleiben daher länger in der Luft, können sich über Luftbewegung im Raum verteilen und das Infektionsgeschehen beeinflussen.

Das Elektroden-Design und die Anordnung der Elektrodeneinrichtung 2 bewirken, dass vor allem die am Infektionsgeschehen maßgeblich beteiligten Aerosol Partikel-Fraktionen zwischen den Kompositelektroden 3, 4 verweilen und dort wirksam dekontaminiert werden können. Beste HEPA Filter scheiden Partikelgrößen zum Beispiel nur bis 0,3 µm ab.

Wie aus FIG 3, FIG 7 und FIG 8 ersichtlich ist, verharrt ein sehr großer Anteil der als gefährlich eingestuften, sogenannten Schwebeaerosol-Partikel, in der spaltartigen Meanderstruktur der Elektrodeneinrichtung 2, wo die UV-C Strahlung generiert wird. Der Anteil der Schwebeaerosol-Partikel kann so unmittelbar mittels der Wirkung der UVC-Strahlen inaktiviert werden. Damit werden sogar Aerosole mit Partikelgrößen durch entstehende Dosisleistungen hochwirksam unter 0,3 µm inaktiviert.

Aufgrund der hohen Photonendichten und der erzwungenen Verweilzeit definierter Partikelfraktionen werden hohe Inaktivierungs-/Dekontaminationsraten erzielt.

Es wird angenommen, dass ein Superspreader bis zu 200.000 2×10^5 Sars-CoV-2 Viruspartikel pro Sekunde und Kubikmeter erzeugt Quelle: Michael Riediker; Dai-Hua Tsai, Estimation of Viral Aerosol Emissions From Simulated Individuals with Asymptomatic to Moderate Coronavirus Disease 2019, JAMA Network doi:10.1001/jamanetworkopen.2020.13807. In einem geschlossenen Raum von zum Beispiel 50 m^3 Volumen können sich so durch eine Person fast 7×10^5-7×10^7 Viruspartikel pro Kubikmeter anreichern. Aufgrund der in der vorliegenden Plasma-Filtereinrichtung 1 erzeugten hohen Photonenanzahl mit weit über 1015 Photonen/s, ist jeder Partikel einer hohen Trefferzahl von UVC-Quanten ausgesetzt. Aufgrund der sehr langen Verweilzeiten der Partikel in den meanderförmigen Entladungsspalten 9 der Elektrodeneinrichtungen 2 wird eine effiziente Inaktivierung ermöglicht. Diese Ergebnisse können in einer hohen Effektivität und Effizienz der vorliegenden Plasma-Filtereinheit führen

Die Bauweise der Plasma-Filtereinrichtung 1 weist Vorteile gegenüber konventionellen Lösungen auf. Aufgrund des Gesamtaufbaus der vorliegenden Plasma-Filtereinrichtung 1 sind laminare Luftströmungen mit sehr geringem Druckverlust 5-6 Pa pro Elektrodeneinrichtung 2, kompletter Aufbau ca. 50 Pa erzielbar.

Durch eine Bereitstellung anwendungsspezifischer Filtergrößen ist eine sehr kompakte und kleine Bauweise von nur wenigen cm, sowie eine Skalierbarkeit für große Filteranlagen im Bereich von Metern möglich, wie in FIG 7 zu erkennen.

Die Plasma-Filtereinrichtung 1 kann hohe Durchströmungsraten von 250 - 300 m³/h bei laminaren Strömungsverhältnissen ermöglichen.

Aufgrund kompakter Bauweise und Standardmaterialien sind die Fertigungskosten gering. Eine durch die Form der Plasma-Filtereinrichtung 1 ermöglichte Flachbauweise lässt eine direkte Integration der Plasma-Filtereinrichtung 1 in zum Beispiel Deckenlüftern zu. Eine Integration der Plasma-Filtereinrichtung 1 in Automotive- und Mobility-Anwendungen ist aufgrund der kompakten Bauweise und einer reduzierten Geräuschemission durch die laminare Luftströmung ermöglicht. Die vorliegende Plasma-Filtereinrichtung 1 kann bestehende Filteranlagen in Flugzeugen ersetzen. Aufgrund der Gewichts- und Bauraum-Einsparung sind neue Design-Möglichkeiten bezüglich der Kabine und tragenden Strukturen gegeben. Durch die Plasma-Filtereinrichtung 1 ist eine direkte Substitution von komplexen Filterkaskaden in Gebäuden möglich.

Die ermöglicht einen reduzierten Einsatz von HEPA Filtereinheiten unterschiedlicher Ausprägung, eine Verbesserung der Energiebilanz, eine Verringerung von Abfall sowie eine Verlängerung und/oder Veränderung von Wartungsintervallen. Aufgrund der kompakten Bauweise ist es möglich, die Plasma-Filtereinrichtung 1 zur Ermöglichung einer hohen elektromagnetischen Verträglichkeit durch Anordnungen gezielt zu schirmen siehe HF- und B-Feld Kompatibilität.

Aufgrund der hohen elektromagnetischen Empfindlichkeit von elektronischen und bildgebenden Komponenten, wie beispielsweise Magnetresonanztomographiegeräten, muss besonderer Wert auf die elektromagnetische Verträglichkeit der Plasmaeinheiten und der Stromversorgungstechnik gelegt werden. Bestehende und marktverfügbare Lösungen müssten hinsichtlich Plasmagenerator und/oder Leistungselektronik sowie der verwendeten Elektrodentechnik aufwendig angepasst werden. In den meisten Fällen ist eine Anpassung nicht möglich.

Um einen vollständig abgeschirmten EMV-Betrieb zu gewährleisten, wird bei der vorliegenden Plasma-Filtereinrichtung 1 eine Struktur von drei gekreuzten oder ungekreuzten, beschichteten Elektrodeneinrichtungen 2 empfohlen, die jeweils durch einen Abstandshalterrahmen getrennt sind. Dadurch entsteht ein Faradayscher Käfig, der die Grundfrequenz von 10 kHz bis zum Abstandsverhältnis der Elektrodenstifte Abstand 15 1-2 mm vollständig abschirmt. Aufgrund der kompakten Bauweise der vorliegenden Elektrodeneinrichtung 2 ist eine Einhausung samt Leistungselektronik und/oder Plasmagenerator in einem zum Beispiel Edelstahlrohr 21 möglich. Eine ausreichende Schirmung ist dadurch gegeben.

## Patentansprüche

1. Plasma-Filtereinrichtung (1), aufweisend
- zumindest eine Elektrodeneinrichtung (2),
**dadurch gekennzeichnet, dass**
- die Elektrodeneinrichtung (2) eine erste plan ausgebildete Kompositelektrode (3) und eine zweite plan ausgebildete Kompositelektrode (4) aufweist, wobei
- die Kompositelektroden (3, 4) der Elektrodeneinrichtung (2) coplanar zueinander in einer Hauptflächenebene (13) der Elektrodeneinrichtung (2) angeordnet und durch einen Entladungsspalt (9) räumlich voneinander getrennt sind, und wobei
- jede der Kompositelektroden (3, 4) ein jeweiliges Elektrodenblech (5, 6) aufweist, das zumindest an einer Grenzfläche des jeweiligen Elektrodenblechs (5, 6) zu dem Entladungsspalt (9) eine jeweilige dielektrische Beschichtung (7, 8) aufweist,
- die Plasma-Filtereinrichtung (1) eine Spannungsquelle (22) aufweist, die dazu eingerichtet ist, eine Wechselspannung an der Elektrodeneinrichtung (2) bereitzustellen, wobei
- die Wechselspannung dazu parameterisiert ist, eine Bildung eines Plasmas (11) durch eine dielektrische Barriereentladung in dem Entladungsspalt (9) hervorzurufen, und
- die Plasma-Filtereinrichtung (1) dazu eingerichtet ist, ein Gas entlang einer Hauptströmungsrichtung (12), welche parallel zu einer Normalen der Hauptflächenebene (13) der Elektrodeneinrichtung (2) ausgerichtet ist, durch den Entladungsspalt (9) zu führen.

2. Plasma-Filtereinrichtung (1) nach Anspruch 1,
**dadurch gekennzeichnet, dass**
jede der Kompositelektroden (3, 4) eine Kammstruktur aufweist, welche Elektrodenfinger aufweist, und die Kammstrukturen der Kompositelektroden (3, 4) der Elektrodeneinrichtung (2) ineinander greifend und durch den Entladungsspalt (9) getrennt zueinander angeordnet sind.

3. Plasma-Filtereinrichtung (1) nach einem der vorhergehenden Ansprüche,
**dadurch gekennzeichnet, dass**
das Elektrodenblech (5, 6) jeder der Kompositelektroden (3, 4) Aluminium, und/oder eine Aluminiumlegierung aufweist.

4. Plasma-Filtereinrichtung (1) nach einem der vorhergehenden Ansprüche,
**dadurch gekennzeichnet, dass**
das Elektrodenblech (5, 6) jeder der Kompositelektroden (3, 4) rostfreien Stahl aufweist.

5. Plasma-Filtereinrichtung (1) nach einem der vorhergehenden Ansprüche,
**dadurch gekennzeichnet, dass**
die dielektrische Beschichtung (7, 8) jeder der Kompositelektroden (3, 4) einen oder mehrere Polymere aufweist.

6. Plasma-Filtereinrichtung (1) nach Anspruch 5,
**dadurch gekennzeichnet, dass**
die dielektrische Beschichtung (7, 8) jeder der Kompositelektroden(3, 4) einen oder mehrere Fluorkunststoffe aufweist.

7. Plasma-Filtereinrichtung (1) nach Anspruch 6,
**dadurch gekennzeichnet, dass**
die dielektrische Beschichtung (7, 8) jeder der Kompositelektroden (3, 4) Polyvinylidendifluorid aufweist.

8. Plasma-Filtereinrichtung (1) nach Anspruch 6 oder 7,
**dadurch gekennzeichnet, dass**
die dielektrische Beschichtung (7, 8) jeder der Kompositelektroden (3, 4) Polytetrafluorethylen aufweist.

9. Plasma-Filtereinrichtung (1) nach einem der vorhergehenden Ansprüche,
**dadurch gekennzeichnet, dass**
die dielektrische Beschichtung (7, 8) jeder der Kompositelektroden (3, 4) Graphitfluorid aufweist.

10. Plasma-Filtereinrichtung (1) nach einem der vorhergehenden Ansprüche,
**dadurch gekennzeichnet, dass**
die dielektrische Beschichtung (7, 8) jeder der Kompositelektroden (3, 4) eine oder mehrere Keramiken aufweist.

11. Plasma-Filtereinrichtung (1) nach Anspruch 10,
**dadurch gekennzeichnet, dass**
die dielektrische Beschichtung (7, 8) jeder der Kompositelektroden (3, 4) Bariumtitanat aufweist.

12. Plasma-Filtereinrichtung (1) nach einem der Ansprüche 10 bis 11,
**dadurch gekennzeichnet, dass**
die dielektrische Beschichtung (7, 8) jeder der Kompositelektroden (3, 4) Kaoloinit aufweist.

13. Plasma-Filtereinrichtung (1) nach einem der Ansprüche 10 bis 12,
**dadurch gekennzeichnet, dass**
die dielektrische Beschichtung (7, 8) jeder der Kompositelektroden (3, 4) einen Blend, umfassend vorgegebene Anteile von Kaoloinit, Aluminiumoxid, Titanoxid, Chromoxid, Bariumtitanat und/oder anderen keramischen Pulvern aufweist.

14. Plasma-Filtereinrichtung (1) nach einem der vorhergehenden Ansprüche,
**dadurch gekennzeichnet, dass**
die Plasma-Filtereinrichtung (1) zumindest zwei der Elektrodeneinrichtungen (2) aufweist,
die Plasma-Filtereinrichtung (1) eine Halteeinrichtung (20) aufweist, welche dazu eingerichtet ist, die zumindest zwei der Elektrodeneinrichtungen (2) in einer vorbestimmten Elektrodenanordnung anzuordnen, wobei die zumindest zwei der Elektrodeneinrichtungen (2) in der vorbestimmten Elektrodenanordnung parallel zueinander ausgerichtet, und entlang der Hauptströmungsrichtung (12) hintereinander und durch einen vorbestimmten Abstand (15) paarweise voneinander getrennt angeordnet sind.

15. Plasma-Filtereinrichtung (1) nach Anspruch 12,
**dadurch gekennzeichnet, dass**
benachbarte Elektrodeneinrichtungen (2) der zumindest zwei Elektrodeneinrichtungen (2) um 90 Grad um die Hauptströmungsrichtung (12) zueinander rotiert ausgerichtet sind.

16. Plasma-Filtereinrichtung (1) nach einem der vorhergehenden Ansprüche,
**dadurch gekennzeichnet, dass**
die Plasma-Filtereinrichtung (1) zumindest einen Staubfilter (16) aufweist, der in einer Hauptströmungsrichtung (12) vor der Elektrodeneinrichtung (2) angeordnet ist.

17. Plasma-Filtereinrichtung (1) nach einem der vorhergehenden Ansprüche,
**dadurch gekennzeichnet, dass**
die Plasma-Filtereinrichtung (1) zumindest einen Aktivkohlefilter (18) aufweist, das in einer Hauptströmungsrichtung (12) hinter der Elektrodeneinrichtung (2) angeordnet ist.

18. Elektrodeneinrichtung (2) für eine Plasma-Filtereinrichtung (1) **dadurch gekennzeichnet, dass** die Elektrodeneinrichtung (2) eine erste plan ausgebildete Kompositelektrode (3) und eine zweite plan ausgebildete Kompositelektrode (4) aufweist, wobei
- die Kompositelektroden (3, 4) der Elektrodeneinrichtung (2) coplanar zueinander in einer Hauptflächenebene (13) der Elektrodeneinrichtung (2) angeordnet und durch einen Entladungsspalt (9) räumlich voneinander getrennt sind, und wobei
- jede der Kompositelektroden (3, 4) ein jeweiliges Elektrodenblech (5, 6) aufweist, das zumindest an einer Grenzfläche des jeweiligen Elektrodenblechs (5, 6) zu dem Entladungsspalt (9) eine jeweilige dielektrische Beschichtung (7, 8) aufweist,

19. Verfahren zum Betreiben einer Plasma-Filtereinrichtung (1), **dadurch gekennzeichnet, dass**
die Plasma-Filtereinrichtung (1) zumindest eine Elektrodeneinrichtung (2) aufweist, wobei die Elektrodeneinrichtung (2) eine erste plan ausgebildete Kompositelektrode (3) und eine zweite plan ausgebildete Kompositelektrode (4) aufweist, wobei
- die Kompositelektroden (3, 4) der Elektrodeneinrichtung (2) coplanar zueinander in einer Hauptflächenebene (13) der Elektrodeneinrichtung (2) angeordnet und durch einen Entladungsspalt (9) räumlich voneinander getrennt sind, und wobei
- jede der Kompositelektroden (3, 4) ein jeweiliges Elektrodenblech (5, 6) aufweist, das zumindest an einer Grenzfläche des jeweiligen Elektrodenblechs (5, 6) zu dem Entladungsspalt (9) eine jeweilige dielektrische Beschichtung (7, 8) aufweist,
wobei durch
eine Spannungsquelle (22) der Plasma-Filtereinrichtung (1), eine Wechselspannung an der Elektrodeneinrichtung (2) bereitgestellt wird, wodurch ein Plasma (11) durch eine dielektrische Barriereentladung in dem Entladungsspalt (9) hervorgerufen wird, und
durch die Plasma-Filtereinrichtung (1), ein Gas entlang einer Hauptströmungsrichtung (12), welche parallel zu einer Normalen der Hauptflächenebene (13) der Elektrodeneinrichtung (2) ausgerichtet ist, durch den Entladungsspalt (9) geführt wird.
